# EUROPEAN PATENT APPLICATION

(11) **EP 4 710 851 A1**
(43) Date of publication of application: **18.03.2026**
(21) Application number: 25192936.0
(22) Date of filing: 30.07.2025
(51) Int. Cl.: A61B 5/055, A61B 5/107, A61B 5/00, A61B 6/04, G01R 33/28

(54) **PROJECTION CONTROL DEVICE, OPERATION METHOD OF PROJECTION CONTROL DEVICE, AND OPERATION PROGRAM OF PROJECTION CONTROL DEVICE**

(30) Priority: 22.08.2024 JP 2024141208
(71) Applicant: FUJIFILM Corporation, Tokyo 106-8620 (JP)
(72) Inventor: MORIWAKE, Chikako, Tokyo, 106-8620 (JP)
(74) Representative: Dehns Germany Partnerschaft mbB

(57) **Abstract**

A projection control device includes a processor, in which the processor is configured to acquire an optical image of a subject, estimate a position of a liver of the subject based on the optical image, and control a projection apparatus to project a marker representing the estimated position of the liver onto the subject, as a marker representing a position at which a receive coil unit of a magnetic resonance imaging apparatus is attached.

## Description

### BACKGROUND OF THE INVENTION

### 1. Field of the Invention

The present disclosure relates to a projection control device, an operation method of a projection control device, and an operation program of a projection control device.

### 2. Description of the Related Art

A magnetic resonance imaging apparatus (hereinafter referred to as MRI apparatus) irradiates a subject disposed in an imaging region with a high-frequency radio frequency (RF) pulse to capture an image representing physical or chemical properties of the subject by using a nuclear magnetic resonance phenomenon (hereinafter referred to as NMR phenomenon), which occurs at a time of the irradiation, and is used particularly for medical purposes.

In a case where hydrogen atoms in the subject in a state of being aligned in a static magnetic field are irradiated with the RF pulse, the hydrogen atoms absorb energy from the RF pulse and are excited. In a case where the high-frequency pulse is stopped, the hydrogen atoms in the excited state attempt to return to the original alignment state and thus release the absorbed energy in the returning process. The above is an NMR phenomenon, and the hydrogen atoms generate a magnetic resonance signal, which is a free induction decay (FID) signal, according to the released energy. The MRI apparatus captures an image of a structure in the subject based on the received FID signal. Since the FID signal is a very weak signal, in a case where the MRI apparatus performs the imaging, a receive coil unit that receives the FID signal is attached to a position corresponding to a portion whose image is desired to be captured in the subject (for example, JP2024-082179A).

### SUMMARY OF THE INVENTION

Since the receive coil unit to be attached to the subject is attached and detached for each imaging and is carried, there is a demand for miniaturization. However, in a case where the miniaturization is performed, a range covered by the receive coil unit is narrowed, and thus there is a problem that an allowable range of misregistration of an attachment position of the receive coil unit is narrowed.

This problem is particularly remarkable in a case where a liver is imaged. Since the liver is an organ whose position changes relatively significantly depending on a physique of the subject, it is difficult to figure out the position of the liver from the outside of the body. In a case where a small receive coil unit is used, a degree of the misregistration that is allowed in a large receive coil unit is not allowed, and thus the attachment position may deviate from the position of the liver. In a case where the receive coil unit is not attached to an appropriate position, an image of a target portion cannot be captured obviously, and thus re-imaging is required.

An example of a solution to the above problem includes a measure of understanding the position of the liver of the subject in advance by radiography before MRI imaging. However, this measure is difficult to be employed due to a disadvantage that the subject is exposed to radiation, in addition to complication of an apparatus configuration.

A technique of the present disclosure provides a projection control device, an operation method of a projection control device, and an operation program of a projection control device capable of guiding an appropriate attachment position of a receive coil unit without performing radiography in a case where an MRI apparatus images a liver.

A projection control device according to the technique of the present disclosure comprises a processor, in which the processor is configured to acquire an optical image of a subject, estimate a position of a liver of the subject based on the optical image, and control a projection apparatus to project a marker representing the estimated position of the liver onto the subject, as a marker representing a position at which a receive coil unit of a magnetic resonance imaging apparatus is attached.

Further, the processor may be configured to derive a degree of obesity of the subject based on the optical image, and estimate the position of the liver according to the derived degree of obesity.

The processor may be configured to derive the degree of obesity based on a first evaluation value that is defined as a ratio, to a body thickness, of a length from a shoulder to a waist in a body axis direction of the subject.

The processor may be configured to derive the degree of obesity based on a second evaluation value that is defined as a ratio of a body thickness at a position of a navel to an average value of a body thickness in a body portion, in addition to the first evaluation value.

The processor may be configured to estimate the position of the liver of the subject using reference information in which a correspondence relationship between the degree of obesity and the position of the liver is stored in advance.

The optical image may include a front image obtained by imaging the subject from a front and a side image obtained by imaging the subject from a side.

The optical image may include two front images having a parallax, which are obtained by imaging the subject from a front.

The processor may be configured to estimate the position of the liver of the subject using a machine learning model that receives the optical image as an input and outputs the position of the liver of the subject.

An operation method of a projection control device according to the technique of the present disclosure is an operation method of a projection control device including a processor, the operation method executed by the processor comprising acquiring an optical image of a subject, estimating a position of a liver of the subject based on the optical image, and controlling a projection apparatus to project a marker representing the estimated position of the liver onto the subject, as a marker representing a position at which a receive coil unit of a magnetic resonance imaging apparatus is attached.

An operation program of a projection control device according to the technique of the present disclosure is an operation program of a projection control device including a processor, the operation program causing the processor to execute a process comprising acquiring an optical image of a subject, estimating a position of a liver of the subject based on the optical image, and controlling a projection apparatus to project a marker representing the estimated position of the liver onto the subject, as a marker representing a position at which a receive coil unit of a magnetic resonance imaging apparatus is attached.

According to the technique of the present disclosure, in a case where the MRI apparatus images the liver, it is possible to guide the appropriate attachment position of the receive coil unit without performing radiography.

### BRIEF DESCRIPTION OF THE DRAWINGS

Fig. 1 is a diagram showing an example of a schematic configuration of an MRI apparatus.
Fig. 2 is a diagram showing an example of a state in which a receive coil unit is attached to a subject.
Fig. 3 is a diagram showing an example of a function of a processor.
Fig. 4 is a diagram showing an example of a relationship between a physique and a liver position.
Fig. 5 is a table showing an example of reference information indicating a correspondence relationship between a degree of obesity and the liver position.
Fig. 6 is a diagram showing a relative positional relationship between a spine and the liver position.
Fig. 7 is a table showing an example of a first evaluation value of the degree of obesity.
Fig. 8 is an example of a flowchart showing an overall processing procedure of projection processing.
Fig. 9 is an example of a flowchart showing a processing procedure for deriving the degree of obesity.
Fig. 10 is a diagram showing an example in which a spine length is measured from an optical image.
Fig. 11 is a diagram showing an example in which a body thickness is measured from an optical image.
Fig. 12 is a diagram showing an example of a state in which a marker of the liver is projected onto the subject.
Figs. 13A and 13B are diagrams for describing a second evaluation value.
Fig. 14 is an example of reference information indicating a relationship between the first and second evaluation values and the degree of obesity.
Fig. 15 is a diagram showing an example in which the degree of obesity is derived using a machine learning model.
Fig. 16 is a diagram showing an example in which a stereo camera is used.

### DESCRIPTION OF THE PREFERRED EMBODIMENTS

### First embodiment

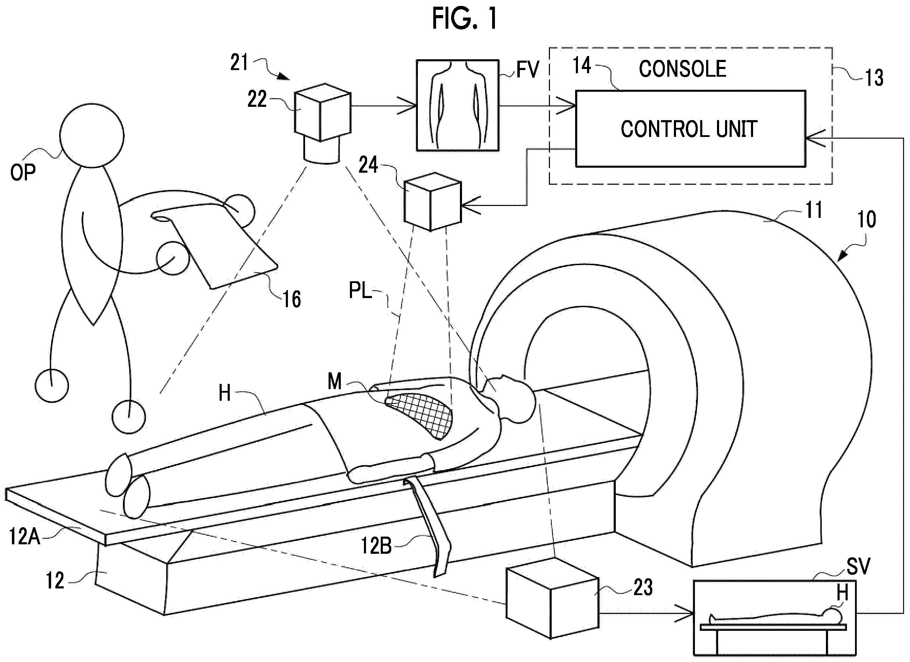

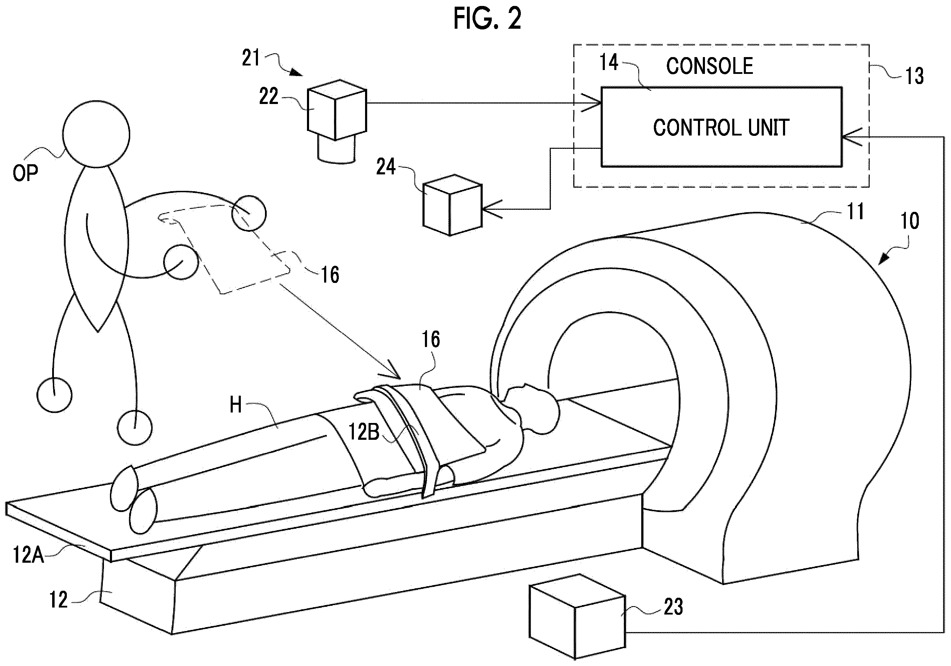

Figs. 1 and 2 are schematic diagrams showing an example of an MRI apparatus 10. The MRI apparatus 10 is, for example, a closed type having a cylindrical bore 11, and comprises a bed 12 and a console 13 in addition to the bore 11. As is well known, the bore 11 is provided with an opening portion through which a subject H can enter, and a static magnetic field magnet for generating a static magnetic field, a gradient magnetic field coil for generating a gradient magnetic field, an RF transmission coil for transmitting an RF pulse, and the like are built in the bore 11. The bed 12 has a top plate 12A on which the subject H in a decubitus posture can be placed. The top plate 12A can enter the opening portion of the bore 11 by sliding movement in a horizontal direction. Further, the bed 12 is provided with a belt 12B for fixing a receive coil unit 16 to be attached to the subject H. The console 13 has a control unit 14 that controls each part of the MRI apparatus 10.

The receive coil unit 16 receives an FID signal generated by hydrogen atoms, which are excited by irradiation of the RF pulse in the subject H. The receive coil unit 16 is configured of a plurality of receive coils and an exterior material that accommodates the plurality of receive coils in a state of being arranged. The exterior material has flexibility, and is curved along a body surface of the subject H. The body surface refers to an outer surface of the subject H in a clothed state. Since the exterior material is curved along the body surface, the receive coil can be disposed close to the body surface. In the related art, the exterior material has been mainly configured to be curved as a whole by connecting a plurality of hard parts made of a hard resin material to each other in a movable manner, but in recent years, there has been an increase in the exterior materials made of a flexible fabric. The receive coil unit 16 of the present example shown in Figs. 1 and 2 shows, for example, a type in which the exterior material is made of the flexible fabric. The receive coil unit 16 is attached to a body of the subject H, and has, for example, a rectangular planar shape.

As shown in Fig. 2, the receive coil unit 16 is attached to a position corresponding to an imaging portion whose image is desired to be captured in the subject H, by an operator OP, such as a medical radiologist. In a case where the imaging portion is a liver, the receive coil unit 16 is attached to a position corresponding to the liver. The receive coil unit 16 is attachable and detachable to and from the subject H, and is attached, for example, to cover a front side of the subject H in a supine posture.

The receive coil is built in the top plate 12A of the bed 12, and the FID signal released from a rear side of the subject H is received by the receive coil in the top plate 12A.

Further, the MRI apparatus 10 comprises a projection system 21. The projection system 21 estimates a position of the liver of the subject H, and projects a marker M representing the estimated position of the liver onto the body surface of the subject H as shown in Fig. 1. The projection system 21 comprises optical cameras 22 and 23, a projection apparatus 24, and the control unit 14. The control unit 14 serves as both a control unit of the MRI apparatus 10 and a control unit of the projection system 21. Of course, the control units may be configured separately.

The optical cameras 22 and 23 optically image the subject H. The optical cameras 22 and 23 capture an optical image of the subject H, which is generated by visible light, for example. The optical cameras 22 and 23 each have an imaging optical system that forms an image of light of the subject H and an image sensor that outputs the formed optical image as an electrical image signal. The image sensor is, for example, a complementary metal oxide semiconductor (CMOS) image sensor or a charge coupled device (CCD) image sensor.

The optical camera 22 images, from a front, the subject H in the supine posture on the bed 12, and outputs, to the control unit 14, a front image FV as the optical image of the subject H. The optical camera 22 is disposed, for example, on a ceiling of an imaging room in which the MRI apparatus 10 is installed.

The optical camera 23 images, from a side, the subject H in the supine posture on the bed 12, and outputs, to the control unit 14, a side image SV as the optical image of the subject H. The optical camera 23 is disposed, for example, on a wall of the imaging room or on a stand installed on a side of the bed 12.

The projection apparatus 24 projects projection light PL representing the marker M toward the subject H to project the marker M onto the body surface of the subject H. The projection apparatus 24 includes a display unit that displays the marker M and a projection optical system that projects the projection light PL representing the marker M displayed on the display unit. The display unit is, for example, a transmissive type configured by a combination of a liquid crystal display (LCD) and a light source, or a reflective type configured by a combination of digital light processing (DLP) and a light source.

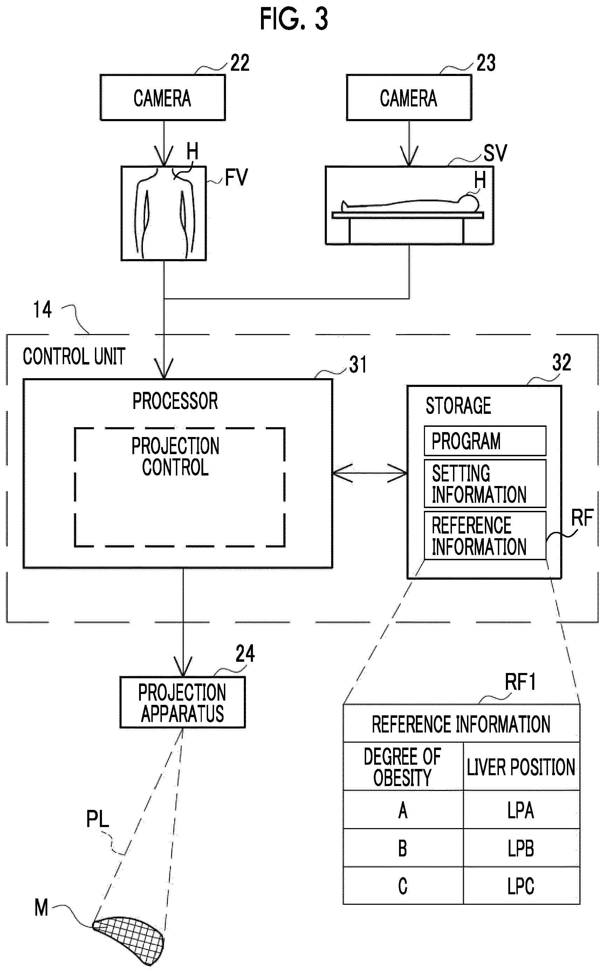

As shown in Fig. 3, the control unit 14 comprises a processor 31 and a storage 32. The processor 31 is configured of, for example, a central processing unit (CPU) and a memory, such as a random access memory (RAM), and executes a program loaded in the memory to execute projection control of the projection system 21. The control unit 14 is an example of "projection control device" according to the technique of the present disclosure. The storage 32 is a data storage that stores a program, setting information, reference information RF, and the like, and is configured of a hard disk drive, a solid state drive, a non-volatile memory, and the like. The program includes an application program causing the processor 31 to function as the control unit 14. The program is an example of "operation program" according to the technique of the present disclosure.

The projection control executed by the processor 31 includes processing of performing image analysis on the optical images of the front image FV and the side image SV acquired from the optical cameras 22 and 23 to estimate the position of the liver of the subject H, and processing of determining a projection position corresponding to the estimated position of the liver and projecting the marker M to the projection position. The reference information RF stored in the storage 32 is information referred to by the processor 31 in a case where the projection control is executed. The reference information RF includes reference information RF1 indicating a correspondence relationship between a physique (degree of obesity in the example of Fig. 3) of the subject H and the liver position, and the like. The reference information RF will be described below. Further, the marker M is an image imitating a form of the liver, for example. An image to be used for the marker M is included in the setting information.

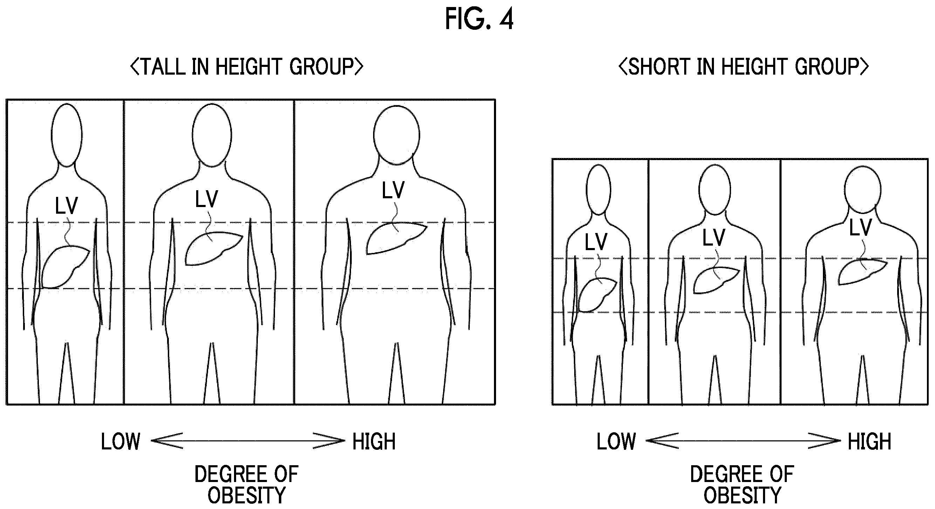

Fig. 4 shows how a position of a liver LV changes according to a person's physique. The position of the liver LV changes in a body axis direction according to the degree of obesity, regardless of tall or short in height. Specifically, the liver LV shifts to a head part side as the degree of obesity is higher, and the liver LV shifts to a foot side as the degree of obesity is lower. It is presumed that this is because, in a case where fat in an abdomen is large, the liver LV is pushed up to the head part side by the fat.

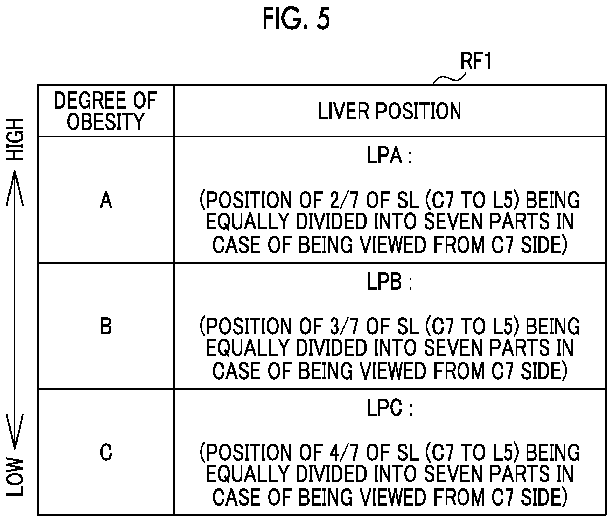

The reference information RF1 shown in Fig. 5 shows an example of a result of statistical analysis of the correspondence relationship between the degree of obesity and the position of the liver LV. In the reference information RF1, a level of the degree of obesity is classified into three stages of A to C, and the liver position for each level is indicated. The levels of the degree of obesity are high in an order of A, B, and C. LPA, LPB, and LPC representing the liver position correspond to respective levels A, B, and C of the degree of obesity. In the reference information RF1, the liver position is a position represented with reference to a spine.

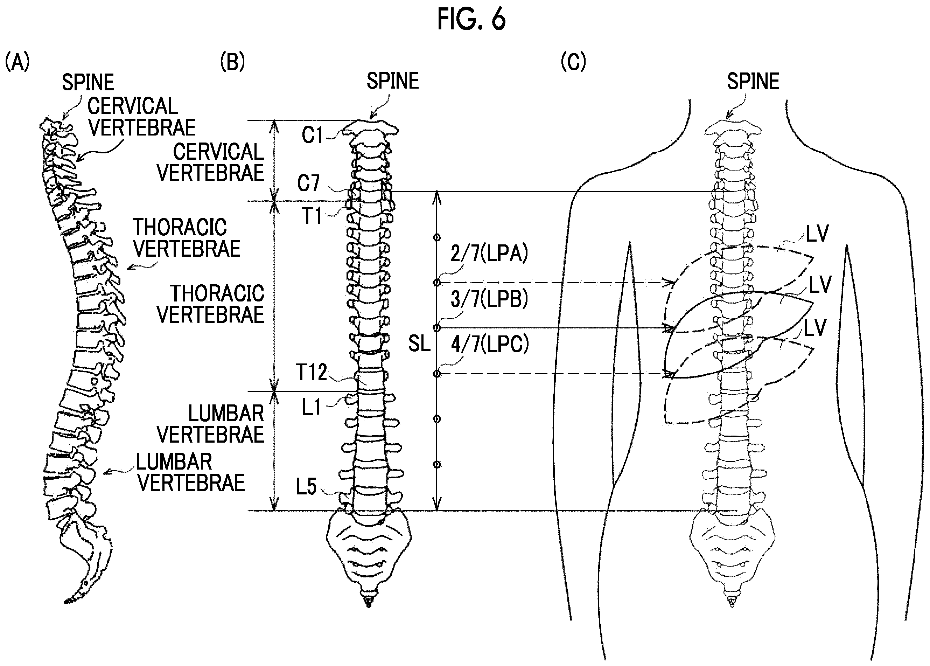

In Fig. 6, (A) of Fig. 6 is a schematic view of the spine as viewed from a side, and (B) of Fig. 6 is a schematic view of the spine as viewed from a front. (C) of Fig. 6 shows the liver positions of LPA to LPC in correspondence with the schematic diagram of the spine. The spine is a bone part extending from a neck to a lower abdomen along the body axis direction, and includes cervical vertebrae corresponding to a position of the neck, thoracic vertebrae corresponding to a position of a chest, and lumbar vertebrae corresponding to a position of a waist, which are arranged in order from the head part side. The cervical vertebrae are configured of seven blocks from a first cervical vertebra C1 to a seventh cervical vertebra C7, which are arranged in order from the head part side. The thoracic vertebrae are configured of twelve blocks from a first thoracic vertebra T1 to a twelfth thoracic vertebra T12, which are arranged in order from the head part side. The lumbar vertebrae are configured of five blocks from a first lumbar vertebra L1 to a fifth lumbar vertebra L5, which are arranged in order from the head part side.

Each of the liver positions LPA to LPC is set based on SL, which is a length from the seventh cervical vertebra C7 to the fifth lumbar vertebra L5 in the spine. Here, SL is not a total length of the spine, but represents a length of a specific range of a part of the spine and is also referred to as a spine length for convenience. In the body axis direction, the seventh cervical vertebra C7 is a position corresponding to a shoulder joint, and the fifth lumbar vertebra L5 is a position corresponding to an iliac crest. In the reference information RF1 shown in Fig. 5, in a case where the degree of obesity is the level A, the liver position is a position of 2/7 of the spine length SL being equally divided into seven parts in a case of being viewed from a side on the seventh cervical vertebra C7. In (C) of Fig. 6, this position is indicated at 2/7 (LPA). Similarly, in a case where the degree of obesity is level B, the liver position is a position of 3/7 of the spine length SL being equally divided into seven parts in a case of being viewed from the side on the seventh cervical vertebra C7. In (C) of Fig. 6, this position is indicated at 3/7 (LPB). Similarly, in a case where the degree of obesity is level C, the liver position is a position of 4/7 of the spine length SL being equally divided into seven parts in a case of being viewed from the side on the seventh cervical vertebra C7. In (C) of Fig. 6, this position is indicated at 4/7 (LPC). In this manner, the liver position is shifted to the head part side as the level of the degree of obesity is higher.

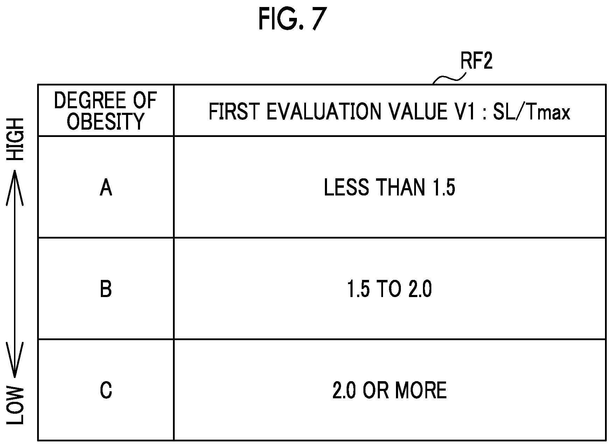

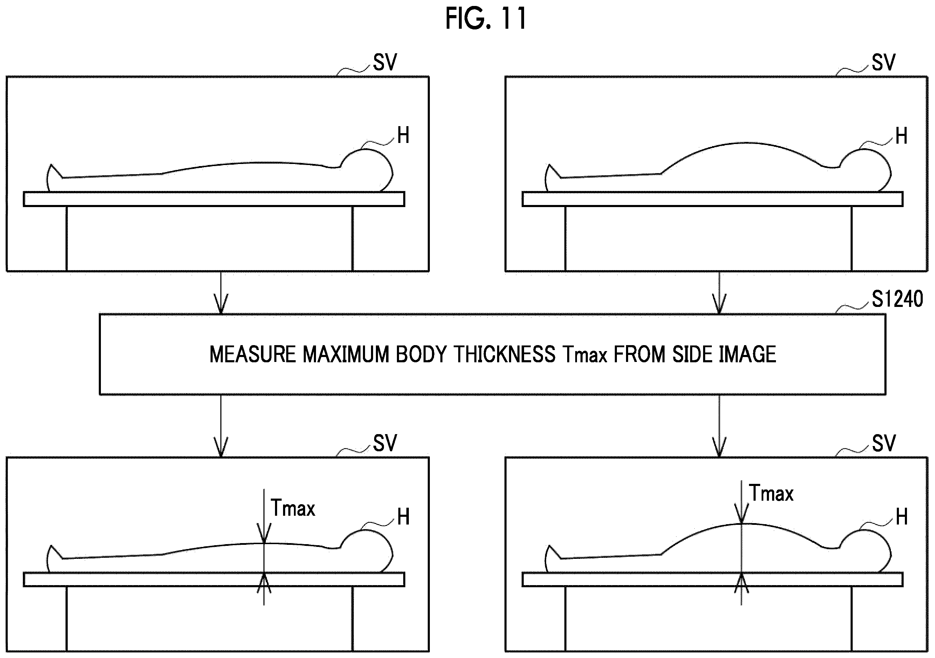

Reference information RF2 shown in Fig. 7 shows definitions of respective levels A to C of the degree of obesity. As shown in the reference information RF2, respective levels A to C are classified based on a value of a first evaluation value V1. The first evaluation value V1 is defined by SL/Tmax. The first evaluation value V1 is an example of an evaluation value of the degree of obesity. As described above, SL is the spine length from the seventh cervical vertebra C7 to the fifth lumbar vertebra L5. Tmax is a maximum value of a body thickness in a section of the spine length SL, and is referred to as a maximum body thickness below (refer to Fig. 11). The degree of obesity of the present example is derived from the first evaluation value V1, which is a ratio of the spine length SL to the maximum body thickness Tmax. The spine length SL is a value that increases according to the height, and the degree of obesity is higher as the value of the maximum body thickness Tmax, which is a denominator, is larger in a case of the same height. In a case where the value of the maximum body thickness Tmax is the same, the degree of obesity is higher as the value of the spine length SL, which is a numerator, is smaller. That is, the level of the degree of obesity is higher as the value of the first evaluation value V1 is smaller. In a case where the first evaluation value V1 is less than 1.5, the degree of obesity is classified as the level A. In a case where the first evaluation value V1 is 1.5 to 2.0, the degree of obesity is classified as the level B. In a case where the first evaluation value V1 is 2.0 or more, the degree of obesity is classified as the level C.

The processor 31 estimates the liver position using the reference information RF1 and the reference information RF2.

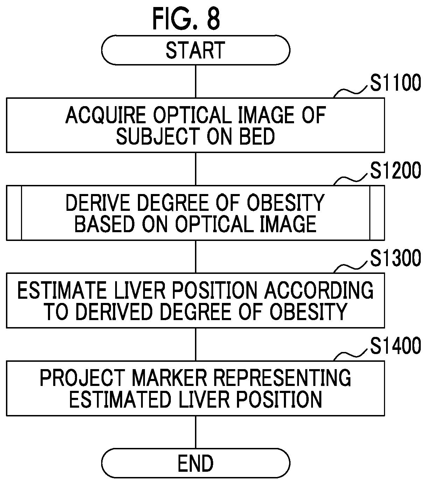

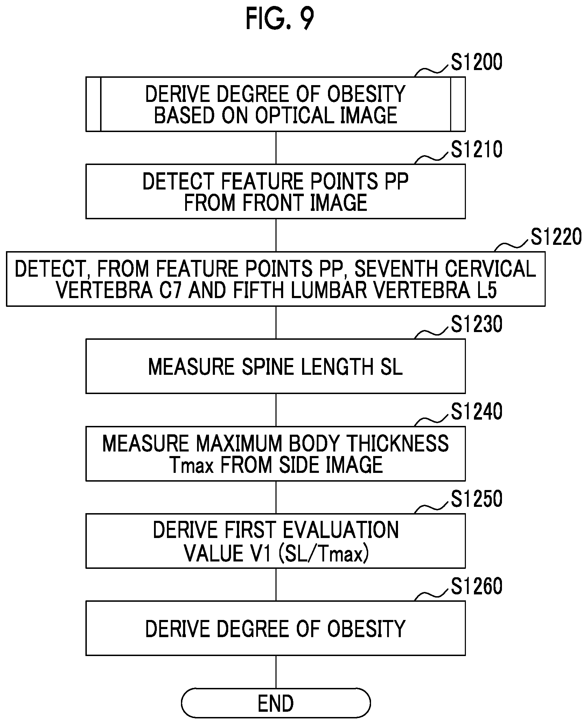

Hereinafter, an action of the above configuration will be described with reference to Figs. 8 to 12. Fig. 8 is a flowchart showing an overall processing procedure of projecting the liver position. Fig. 9 shows sub-steps of step S1200 included in the flowchart shown in Fig. 8.

In a case where the imaging is performed using the MRI apparatus 10, the operator OP first causes the subject H to lie supine on the bed 12. In this state, the top plate 12A of the bed 12 is located outside the opening portion of the bore 11, and the subject H has not entered the opening portion of the bore 11, as shown in Figs. 1 and 2. In order to attach the receive coil unit 16 to the subject H in this state, the operator OP instructs, from the console 13, the projection system 21 to execute the projection processing of the liver position.

As shown in Fig. 8, first, in step S1100, the processor 31 of the projection system 21 causes the optical cameras 22 and 23 to perform the imaging to acquire the optical image, including the front image FV and the side image SV, of the subject H on the bed 12.

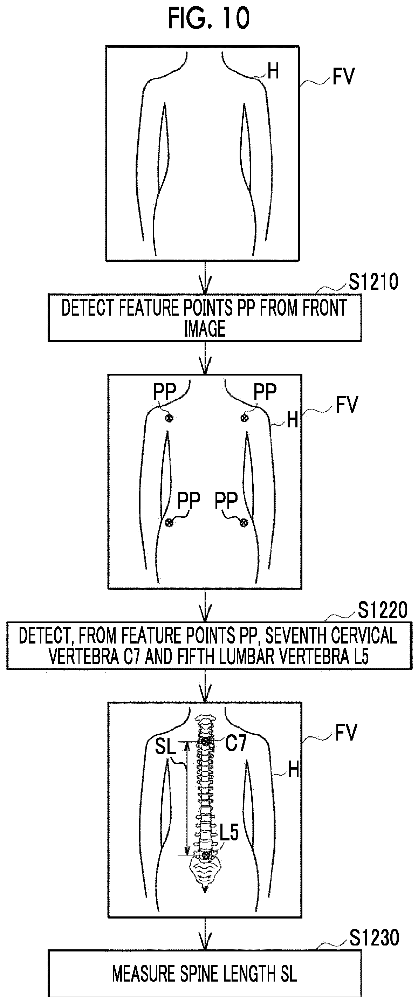

In step S1200, the processor 31 derives the degree of obesity based on the optical image. As shown in Fig. 9, first, in step S1210, the processor 31 detects feature points PP from the front image FV. The feature points PP are detected as shown in Fig. 10, for example. The processor 31 performs, for example, contour extraction of the subject H on the front image FV, and detects the feature points PP corresponding to right and left shoulder joints and right and left iliac crests by a pattern matching method set in advance based on the extracted contour.

A relative positional relationship between the feature points PP and each of the seventh cervical vertebra C7 and the fifth lumbar vertebra L5 is set in advance. As an example, the seventh cervical vertebra C7 is located at a height substantially the same as a height of the feature points PP of the left and right shoulder joints in the body axis direction. The fifth lumbar vertebra L5 is located at a height substantially the same as a height of the feature points PP of the left and right iliac crests in the body axis direction.

In step S1220, the processor 31 detects, from the feature points PP, the seventh cervical vertebra C7 and the fifth lumbar vertebra L5 based on the relative positional relationship with the feature points PP. The spine is shown in the front image FV in Fig. 10, but the display is for convenience for clearly showing positions of the seventh cervical vertebra C7 and the fifth lumbar vertebra L5, and naturally, the spine is not shown in an actual front image FV.

In step S1230, the processor 31 measures, as the spine length SL, a distance between the detected seventh cervical vertebra C7 and fifth lumbar vertebra L5.

After step S1230, the processor 31 proceeds to step S1240, as shown in Fig. 9. In step S1240, the processor 31 measures the maximum body thickness Tmax from the side image SV.

Fig. 11 conceptually shows a state in which the maximum body thickness Tmax is measured based on the side image SV. The processor 31 extracts the contour of the subject H from the side image SV, and measures, as the body thickness, a distance between upper and lower contour lines. Then, the processor 31 records a value at which the body thickness is maximized as the maximum body thickness Tmax. In Fig. 11, the side images SV on a left side show the subject H having a relatively low degree of obesity, and the side images SV on a right side show the subject H having a relatively high degree of obesity. The subject H on the right side having a high degree of obesity has a thicker body thickness in a body portion overall and a thicker maximum body thickness Tmax than the subject H on the left side.

After step S1240, the processor 31 proceeds to step S1250, as shown in Fig. 9. In step S1250, the processor 31 derives, as the first evaluation value V1, the ratio of the measured spine length SL to maximum body thickness Tmax. In step S1260, based on the reference information RF2 shown in Fig. 7, the processor 31 derives the level of the degree of obesity based on the derived value of the first evaluation value V1.

After step S1200, the processor 31 proceeds to step S1300, as shown in Fig. 8. In step S1300, based on the reference information RF1 shown in Fig. 5, the processor 31 estimates the liver positions LPA to LPC according to the derived respective levels A to C of the degree of obesity.

In step S1400, the processor 31 projects the marker M representing the estimated liver position. A relative relationship between positions of the optical camera 22 and the projection apparatus 24 is known. Further, a distance between each of the optical camera 22 and the projection apparatus 24 and the top plate 12A on which the subject H lies supine is also known. Further, the processor 31 can understand, from the distance to the top plate 12A, a distance to the body surface of the subject H onto which the marker M is projected by subtracting the body thickness of the subject H from the side image SV. Further, the processor 31 can also understand a body width of the subject H from the front image FV captured by the optical camera 22. The processor 31 derives, based on these pieces of information, an actual projection position on the body surface of the subject H corresponding to the estimated liver position and a projection size of the marker M. The processor 31 controls the projection apparatus 24 based on the projection position and the projection size decided in this manner.

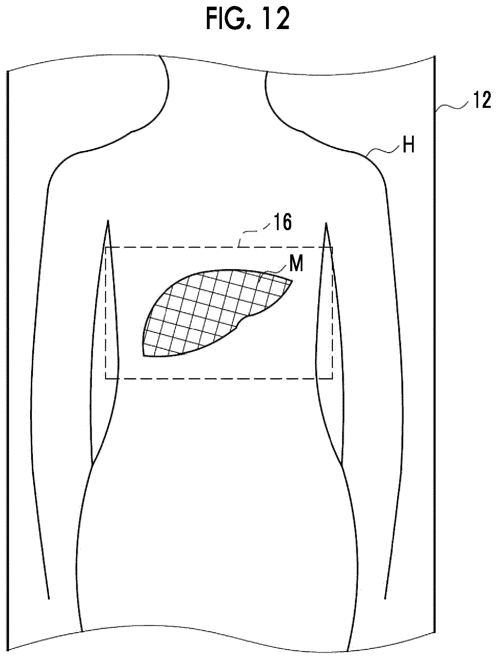

As shown in Fig. 12 (also refer to Fig. 1), the processor 31 controls the projection apparatus 24 according to the derived projection position and projection size to project the marker M to a position corresponding to the liver position of the subject H on the bed 12. As shown in Fig. 12 (also refer to Fig. 2), the operator OP positions the attachment position of the receive coil unit 16 in the body axis direction with the marker M as a guide, and attaches the receive coil unit 16 to the subject H. The MRI apparatus 10 performs the imaging in a state in which the receive coil unit 16 is attached to an appropriate position in this manner.

As described above, the projection control device according to the technique of the present disclosure shown as the control unit 14 as an example comprises the processor 31, and the processor 31 acquires the optical image (the front image FV and the side image SV as an example) of the subject H and estimates the position of the liver of the subject H based on the optical image. The processor 31 controls the projection apparatus 24 to project, onto the subject H, the marker M representing the estimated position of the liver LV, as a marker representing the position to which the receive coil unit 16 of the MRI apparatus 10 is attached. Accordingly, in a case where the MRI apparatus 10 images the liver LV, it is possible to guide the appropriate attachment position of the receive coil unit 16 without performing radiography.

A width of the liver LV that changes according to the physique of the subject H in the body axis direction is large as compared with other organs, and thus is difficult to figure out the position of the liver LV from the appearance. For this reason, it may be difficult to appropriately attach the receive coil unit 16 to a position corresponding to the position of such a liver LV. Such a difficulty is particularly remarkable in a case where the receive coil unit 16 is miniaturized (specifically, length in the body axis direction is shortened). According to the technique of the present disclosure, since the marker M representing the position of the liver LV can be projected onto the body surface of the subject H, the operator OP can understand the position of the liver LV at a glance. Therefore, it is easy to find the appropriate attachment position of the receive coil unit 16.

Further, in the above embodiments, the processor 31 derives the degree of obesity of the subject H based on the optical image, and estimates the position of the liver LV according to the derived degree of obesity. As shown in Fig. 4, since the degree of obesity is correlated with the position of the liver LV, the estimation of the position of the liver according to the degree of obesity is effective for improving estimation accuracy.

Further, in the above embodiments, the processor 31 derives the degree of obesity based on the first evaluation value V1 defined as a ratio, to a body thickness (for example, maximum body thickness Tmax), of a length (for example, spine length SL) from a shoulder (for example, shoulder joint) to a waist (for example, iliac crest) in the body axis direction of the subject H, and estimates the position of the liver LV of the subject H based on the derived degree of obesity. Such a first evaluation value V1 is an evaluation value based on the statistical analysis of the person's physique, and thus has high validity.

Further, in the above embodiments, the processor 31 estimates the position of the liver LV of the subject H, using reference information (for example, reference information RF1) in which the correspondence relationship between the degree of obesity and the position of the liver LV is stored in advance. Since the reference information RF1 is used, operation processing may be simplified as compared with a case where a function is used.

Further, in the above embodiments, the optical image includes the front image FV obtained by imaging the subject H from the front and the side image SV obtained by imaging the subject H from the side. Accordingly, it is possible to derive the degree of obesity based on the body thickness measured in the side image SV as described above. Therefore, it is possible to accurately understand the degree of obesity, as compared with, for example, a case where the degree of obesity is derived using only information (for example, body width) that can be measured from the front image FV.

### Second embodiment

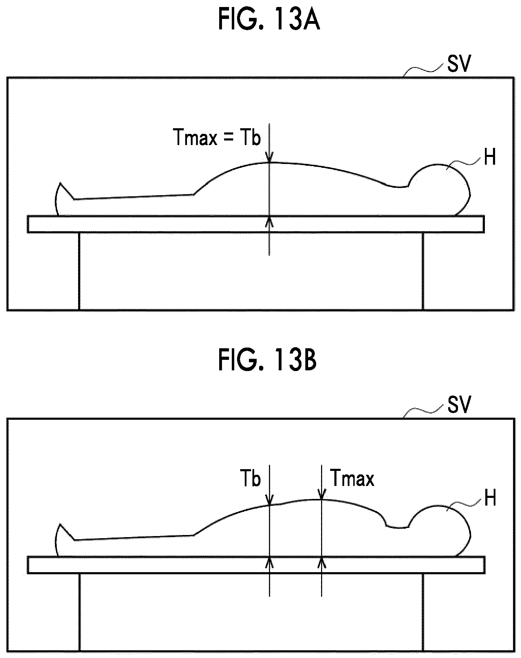

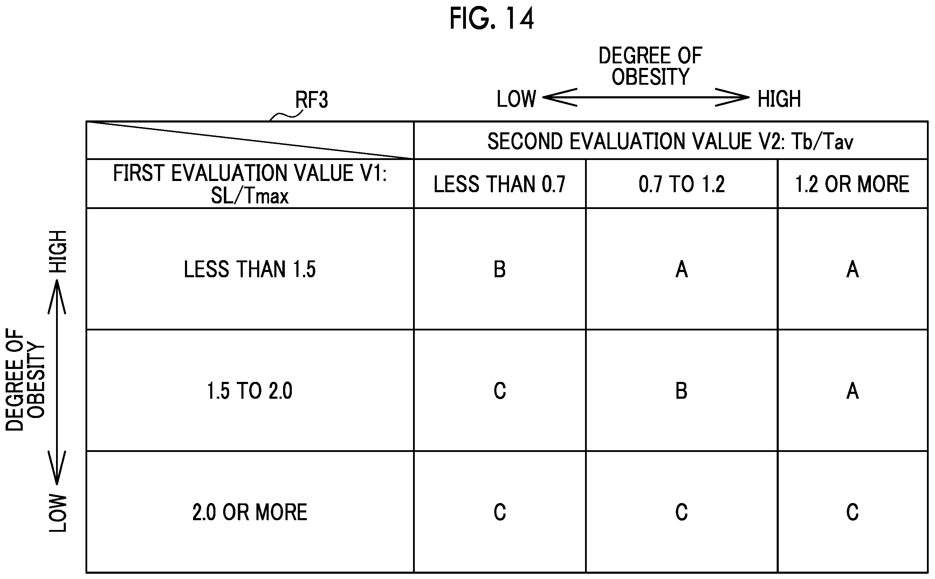

A second embodiment shown in Figs. 13A, 13B, and 14 is an example in which a second evaluation value V2 is used, in addition to the first evaluation value V1, as the evaluation value for deriving respective levels A to C of the degree of obesity. As shown in Fig. 14, the second evaluation value V2 is defined by Tb/Tav. Here, Tb is a body thickness at a position of a navel, Tav is an average value of the body thicknesses in the section of the spine length SL as an example, and the second evaluation value V2 is a ratio of Tb to Tav.

Even in a case where the maximum body thickness Tmax is the same, the position of the liver LV may be different depending on muscle attachment. As shown in Figs. 13A and 13B, in the subject H of Fig. 13A in an upper part, the body thickness Tb at the position of the navel matches the maximum body thickness Tmax. On the other hand, in the subject H of Fig. 13B in a lower part, the body thickness Tb at the position of the navel does not match the maximum body thickness Tmax, and the maximum body thickness Tmax is shifted to the head part side. Fig. 13A shows the subject H having a normal obesity type with a large amount of fat, and Fig. 13B shows the subject H having a body type of an athlete with developed chest muscles, such as a hammer thrower or a wrestler. In a case of the normal obesity type in Fig. 13A, the liver position is shifted toward the head part side due to compression of the fat in the abdomen as shown in Fig. 4. On the other hand, in a case of the muscular case as shown in Fig. 13B, the shift of the liver LV to the head part side is considered to be suppressed by the chest muscles, and thus the liver position often remains on an abdomen side without being shifted toward the head part side as shown in Fig. 13A. For this reason, there may be a case where the degree of obesity cannot be evaluated by distinguishing the normal obesity type and the muscular type as shown in Figs. 13A and 13B, only with the first evaluation value V1 in which only the maximum body thickness Tmax is simply used. Thus, the second evaluation value V2 is considered in addition to the first evaluation value V1.

Reference information RF3 shown in Fig. 14 is a table in which the first evaluation value V1 is assigned to a vertical axis and the second evaluation value V2 is assigned to a horizontal axis, and is a derivation table for deriving respective levels A to C of the degree of obesity with a matrix of the first evaluation value V1 and the second evaluation value V2, as an example. As shown in Fig. 13A, in a typical obesity type, there are many cases where the fat accumulates in the abdomen and the maximum body thickness Tmax is exhibited near the position of the navel. For this reason, the degree of obesity is evaluated to be higher as a value of the body thickness Tb at the position of the navel with respect to the average body thickness Tav is higher. In the reference information RF3, the second evaluation value V2 is classified into three stages of less than 0.7, 0.7 to 1.2, and 1.2 or more, as an example. The degree of obesity is evaluated to be higher as the first evaluation value V1 is smaller and the second evaluation value V2 is larger. In the reference information RF3, the closer to an upper right, the closer to the level A, and the closer to a lower left, the closer to the level C.

The processor 31 estimates the liver position by using the reference information RF3 and the reference information RF1. Accordingly, it is possible to evaluate the degree of obesity in consideration of the difference in the subject H as shown in Figs. 13A and 13B.

### Modification Example 1: Use of machine learning model

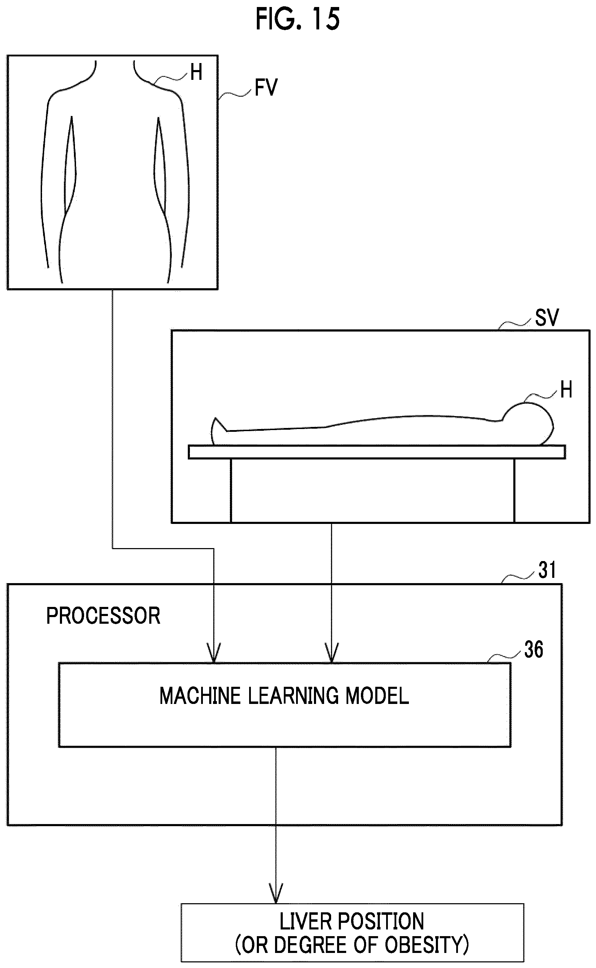

As shown in Fig. 15, a machine learning model 36 may be used for estimating the liver position. The machine learning model 36 outputs the liver position of the subject H with, as inputs, the front image FV and the side image SV, as an example. The machine learning model 36 is, for example, a model that has been trained with, as training data, a pair of the optical images of the front image FV and the side image SV and the liver position serving as correct answer data. Further, the machine learning model 36 may output respective levels A to C of the degree of obesity, instead of the liver position. In this case, the processor 31 decides the liver position according to respective levels A to C of the degree of obesity, based on the reference information RF1.

### Modification Example 2: Use of stereo camera

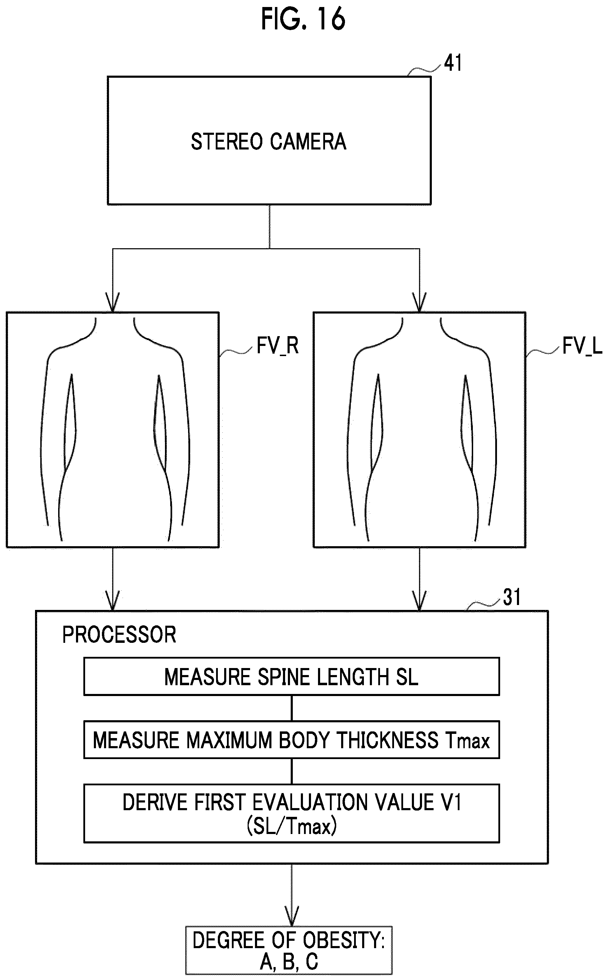

Further, as shown in Fig. 16, an optical image may be acquired by using a stereo camera 41. The optical image is two front images FV_R and FV_L having parallax, which are obtained by imaging the subject H from the front. In a case where there are such two front images FV_R and FV_L, it is possible to measure depth information including the distance to the body surface of the subject H. The processor 31 performs the image analysis on these front images FV_R and FV_L to measure the spine length SL, measure the maximum body thickness Tmax, and derive the first evaluation value V1, as an example. In a case where the stereo camera 41 is used in this manner, the side image SV is not necessary.

The stereo camera 41 is, for example, an optical camera in which a plurality of optical systems, which are disposed at spacings in a single housing, are built. Of course, two cameras having one optical system, such as the optical camera 22, may be prepared, and two optical cameras 22 may be disposed at spacings to function as the stereo camera 41.

A distance-measuring sensor may be used to understand a distance for measuring the body thickness of the subject H. Examples of the distance-measuring sensor include laser imaging detection and ranging or light detection and ranging (LIDAR) and a time-of-flight (TOF) camera. Further, a distance-measuring sensor using ultrasonic waves may be used. Distance images that can be acquired by these distance-measuring sensors may be used, in addition to the optical images such as the front image FV. The distance image acquired by the LIDAR or the TOF camera is included in the optical image. In a case where the contour of the subject H can be detected and the spine length SL can also be measured in such a distance image, the distance image may be used instead of the optical image.

Further, in the above embodiments, an example has been described in which the form of the liver is imitated as the marker M, but the present invention is not limited to this form. Since any mark can be employed as long as the liver position is indicated, the marker M may be, for example, a line indicating a range in which the liver is present in the body axis direction, as in a broken line shown in Fig. 4. Of course, the marker M imitating the form of the liver is considered to be easily recognized by the operator OP in a more intuitive manner, which is preferable.

Further, an example has been described in which the ceiling and the wall of the imaging room are used as the installation positions of the optical cameras 22 and 23, but the optical cameras 22 and 23 may be provided on an interior wall of the opening portion of the bore 11, for example. However, since the attachment of the receive coil unit 16 is performed in a state in which the top plate 12A is disposed outside the opening portion of the bore 11, it is preferable that the optical cameras 22 and 23 are disposed at positions where the imaging can be performed outside the opening portion of the bore 11, such as the ceiling and the wall of the imaging room.

Further, in the above embodiments, various types of hardware described below can be used as hardware of the processor 31. The various types of hardware include, in addition to a CPU that is a general-purpose processor functioning as various processing portions by executing software (a program), a programmable logic device (PLD) such as a field-programmable gate array (FPGA) that has a circuit configuration changeable after manufacture, a dedicated electric circuit such as an application specific integrated circuit (ASIC) that has a circuit configuration dedicatedly designed to execute specific processing, and the like.

Further, the various types of processing may be executed by one of the various types of hardware or may be executed by a combination of two or more pieces of hardware of the same type or different types (for example, a plurality of FPGAs and a combination of a CPU and an FPGA). Further, a plurality of processing portions may be configured of one piece of hardware. Examples of the plurality of processing portions configured of one piece of hardware include a form of using hardware that implements functions of the whole system including the plurality of processing portions in one integrated circuit (IC) chip, like a system on chip (SOC).

In this manner, the various processing portions are configured of one or more of the various types of hardware.

Furthermore, more specifically, an electric circuit (circuitry) obtained by combining circuit elements, such as semiconductor elements, can be used as various hardware structures.

Further, the technique of the present disclosure extends to a computer-readable storage medium (such as universal serial bus (USB) memory or digital versatile disc (DVD)-read only memory (ROM)) that non-transitorily stores a program, in addition to the program causing the processor 31 to function as the projection control device. Further, the technique of the present disclosure can also be employed for a program and a program product.

From the above description, it is possible to understand the techniques described in the following Supplementary Notes.

### Supplementary Note 1

A projection control device comprising:
a processor,
wherein the processor is configured to:
   acquire an optical image of a subject;
   estimate a position of a liver of the subject based on the optical image; and
   control a projection apparatus to project a marker representing the estimated position of the liver onto the subject, as a marker representing a position at which a receive coil unit of a magnetic resonance imaging apparatus is attached.

### Supplementary Note 2

The projection control device according to Supplementary Note 1,
wherein the processor is configured to:
derive a degree of obesity of the subject based on the optical image; and
estimate the position of the liver according to the derived degree of obesity.

### Supplementary Note 3

The projection control device according to Supplementary Note 2,
wherein the processor is configured to:
derive the degree of obesity based on a first evaluation value that is defined as a ratio, to a body thickness, of a length from a shoulder to a waist in a body axis direction of the subject.

### Supplementary Note 4

The projection control device according to Supplementary Note 3,
wherein the processor is configured to:
derive the degree of obesity based on a second evaluation value that is defined as a ratio of a body thickness at a position of a navel to an average value of a body thickness in a body portion, in addition to the first evaluation value.

### Supplementary Note 5

The projection control device according to any one of Supplementary Notes 2 to 4, wherein the processor is configured to:
estimate the position of the liver of the subject using reference information in which a correspondence relationship between the degree of obesity and the position of the liver is stored in advance.

### Supplementary Note 6

The projection control device according to any one of Supplementary Notes 2 to 5,
wherein the optical image includes a front image obtained by imaging the subject from a front and a side image obtained by imaging the subject from a side.

### Supplementary Note 7

The projection control device according to any one of Supplementary Notes 2 to 6,
wherein the optical image includes two front images having a parallax, which are obtained by imaging the subject from a front.

### Supplementary Note 8

The projection control device according to any one of Supplementary Notes 1 to 7,
wherein the processor is configured to:
estimate the position of the liver of the subject using a machine learning model that receives the optical image as an input and outputs the position of the liver of the subject.

### Supplementary Note 9

An operation method of a projection control device including a processor, the operation method executed by the processor comprising:
acquiring an optical image of a subject;
estimating a position of a liver of the subject based on the optical image; and
controlling a projection apparatus to project a marker representing the estimated position of the liver onto the subject, as a marker representing a position at which a receive coil unit of a magnetic resonance imaging apparatus is attached.

### Supplementary Note 10

An operation program of a projection control device including a processor, the operation program causing the processor to execute a process comprising:
acquiring an optical image of a subject;
estimating a position of a liver of the subject based on the optical image; and
controlling a projection apparatus to project a marker representing the estimated position of the liver onto the subject, as a marker representing a position at which a receive coil unit of a magnetic resonance imaging apparatus is attached.

### Supplementary Note 11

A magnetic resonance imaging apparatus including a projection control device, the projection control device comprising:
a processor,
wherein the processor is configured to:
   acquire an optical image of a subject;
   estimate a position of a liver of the subject based on the optical image; and
   control a projection apparatus to project a marker representing the estimated position of the liver onto the subject, as a marker representing a position at which a receive coil unit of a magnetic resonance imaging apparatus is attached.

The contents described and illustrated above are for detailed description of the parts according to the technique of the present disclosure and are only an example of the technique of the present disclosure. For example, the descriptions regarding the configurations, the functions, the actions, and the effects are descriptions regarding an example of the configurations, the functions, the actions, and the effects of the part according to the technique of the present disclosure. Accordingly, in the contents described and illustrated above, it is needless to say that removal of an unnecessary part, or addition or replacement of a new element may be employed within a range not departing from the gist of the present technique of the present disclosure. In order to avoid complication and easily understand the parts according to the technique of the present disclosure, in the contents described and illustrated above, common technical knowledge and the like that do not need to be described to implement the technique of the present disclosure are not described.

In the present specification, "A and/or B" is identical to "at least one of A or B". That is, "A and/or B" may be only A, only B, or a combination of A and B. In the present specification, the same description regarding "A and/or B" is also applied in a case of expressing three or more items with the expression of "and/or".

In a case where all of the documents, patent applications, and technical standards described in the specification are built into the specification as references, to the same degree as a case where the incorporation of each of the documents, patent applications, and technical standards as references is specifically and individually noted.

## Claims

1. A projection control device comprising:
a processor,
wherein the processor is configured to:
acquire an optical image of a subject;
estimate a position of a liver of the subject based on the optical image; and
control a projection apparatus to project a marker representing the estimated position of the liver onto the subject, as a marker representing a position at which a receive coil unit of a magnetic resonance imaging apparatus is attached.

2. The projection control device according to claim 1,
wherein the processor is configured to:
derive a degree of obesity of the subject based on the optical image; and
estimate the position of the liver according to the derived degree of obesity.

3. The projection control device according to claim 2,
wherein the processor is configured to:
derive the degree of obesity based on a first evaluation value that is defined as a ratio, to a body thickness, of a length from a shoulder to a waist in a body axis direction of the subject.

4. The projection control device according to claim 3,
wherein the processor is configured to:
derive the degree of obesity based on a second evaluation value that is defined as a ratio of a body thickness at a position of a navel to an average value of a body thickness in a body portion, in addition to the first evaluation value.

5. The projection control device according to any one of claims 2 to 4,
wherein the processor is configured to:
estimate the position of the liver of the subject using reference information in which a correspondence relationship between the degree of obesity and the position of the liver is stored in advance.

6. The projection control device according to any one of the preceding claims,
wherein the optical image includes a front image obtained by imaging the subject from a front and a side image obtained by imaging the subject from a side.

7. The projection control device according to any one of the preceding claims,
wherein the optical image includes two front images having a parallax, which are obtained by imaging the subject from a front.

8. The projection control device according to any one of the preceding claims 1,
wherein the processor is configured to:
estimate the position of the liver of the subject using a machine learning model that receives the optical image as an input and outputs the position of the liver of the subject.

9. An operation method of a projection control device including a processor, the operation method executed by the processor comprising:
acquiring an optical image of a subject;
estimating a position of a liver of the subject based on the optical image; and
controlling a projection apparatus to project a marker representing the estimated position of the liver onto the subject, as a marker representing a position at which a receive coil unit of a magnetic resonance imaging apparatus is attached.

10. An operation program of a projection control device including a processor, the operation program causing the processor to execute a process comprising:
acquiring an optical image of a subject;
estimating a position of a liver of the subject based on the optical image; and
controlling a projection apparatus to project a marker representing the estimated position of the liver onto the subject, as a marker representing a position at which a receive coil unit of a magnetic resonance imaging apparatus is attached.
